# FASCICULE DE BREVET EUROPEEN

(11) **EP 1 646 724 B1**
(45) Date de publication et mention de la délivrance du brevet: **07.01.2009**
(21) Numéro de dépôt: 04767758.8
(22) Date de dépôt: 22.07.2004
(51) Int. Cl.: C12P 19/34, C12R 1/865, C12N 15/81, C12N 15/67

(54) **PROCEDE DE PRODUCTION INDUSTRIELLE D'ARN ET SYSTEME UTILE POUR LADITE PRODUCTION**
GROSSTECHNISCHES VERFAHREN ZUR HERSTELLUNG VON RNA UND SYSTEM ZUR DURCHFÜHRUNG DIESES VERFAHRENS
INDUSTRIAL METHOD FOR PRODUCING RNA AND SYSTEM FOR CARRYING OUT SAID METHOD

(30) Priorité: 23.07.2003 FR 0309024
(43) Date de publication de la demande: 19.04.2006
(73) Titulaire: CENTRE NATIONAL DE LA RECHERCHE SCIENTIFIQUE (CNRS), 75794 Paris Cedex 16 (FR); Université Victor Segalen Bordeaux 2, 33076 Bordeaux Cedex (FR)
(72) Inventeur: DI RAGO, Jean-Paul, F-33480 Sainte Hélène (FR); BONNEFOY, Nathalie, F-91190 Gif-sur-Yvette (FR); DUVEZIN-CAUBET, Stéphane, F-33000 Bordeaux (FR)
(74) Mandataire: Vialle-Presles, Marie José
(86) Numéro de dépôt international: PCT/FR2004/001957
(87) Numéro de publication internationale: WO 2005/010194

(56) Documents cités:
- EP-A2- 0 317 209
- US-A- 3 272 714
- ANZIANO P QM BUTOW R A: "Splicing-defective mutants of the yeast mitochondrial COXI gene can be corrected by transformation with a hybrid maturase gene" PROCEEDINGS OF THE NATIONAL ACADEMY OF SCIENCES OF USA, NATIONAL ACADEMY OF SCIENCE. WASHINGTON, US, vol. 88, no. 13, 1 juillet 1991 (1991-07-01), pages 5592-5596, XP002221675 ISSN: 0027-8424
- BONNEFOY N ET AL: "GENETIC TRANSFORMATION OF SACCHAROMYCES CEREVISIAE MITOCHONDRIA" METHODS IN ENZYMOLOGY, ACADEMIC PRESS INC, SAN DIEGO, CA, US, vol. 350, 2001, pages 97-111, XP001179423 ISSN: 0076-6879

## Description

La présente invention est relative à un procédé de production industrielle d'ARN d'intérêt.

La présente invention est également relative à un système utile pour la production industrielle d'ARN d'intérêt.

Il existe en effet un besoin de production en grandes quantités d'ARN d'intérêt pour la production de médicaments (ARN d'interférence ou siRNA), l'étude de la structure des ARNs (cristallisation, RMN, complexes), ainsi que l'analyse de la fonction des gènes *in vitro* en culture cellulaire ou *in vivo,* notamment par inhibition de l'expression de ces gènes avec des siRNA.

Les méthodes habituellement utilisées pour la production d'une séquence spécifique d'ARN sont essentiellement la transcription *in vitro* et la synthèse chimique.

Les réactions de transcription *in vitro* mettent en oeuvre des ARN polymérases de bactériophage (SP6, T7 ou T3). Le rendement de ces réactions de synthèse et les quantités de produit obtenu est en général limité, notamment en raison de facteurs limitants, tels que les concentrations en nucléotides (effet inhibiteur de concentrations en nucléotides > 8 mM, par exemple), les concentrations des différents éléments constituant le milieu réactionnel et notamment la concentration en ions Mg⁺⁺. De manière générale, il est admis que les concentrations en magnésium doivent être en excès dans les réactions de transcription *in vitro.* L'utilisation de pyrophosphatase, en association avec les ions Mg⁺⁺ a également été proposée et est considérée comme améliorant le rendement de la réaction de transcription.

Une autre complication rencontrée dans la synthèse *in vitro* de poly-nucléotides est l'inhibition des polymérases de phages à des concentrations en ions relativement faibles.

Pour éviter ces différents inconvénients, l'utilisation de milieux réactionnels améliorés a été proposée dans le Brevet US 5,256,555, qui décrit l'utilisation d'un milieu réactionnel comprenant des concentrations totales molaires élevées en nucléotides (entre 12 mM et 40 mM), qui étaient précédemment considérées comme des concentrations inhibitrices et une quantité molaire efficace de Mg⁺⁺, qui est sous-saturante eu égard aux concentrations totales molaires en nucléotides, de la pyrophosphatase et des Mg⁺⁺-nucléotides ou des Tris-nucléotides.

Malgré les différentes améliorations proposées, la transcription *in vitro* pour produire des ARN d'intérêt, présente les inconvénients suivants :
- induction de réactions parasites (activité N+1) qui augmentent l'hétérogénéité des produits de transcription et nécessitent une purification poussée de l'ARN ;
- limitation quant à la quantité et à la taille de l'ARN synthétisé ;
- coût de production relativement élevé.

Alors qu'il existe des systèmes cellulaires pour produire des protéines d'intérêt *in vivo,* notamment en cellules de levure, il n'existe pas de système comparable pour produire des ARN d'intérêt.

En effet :
- le Brevet américain US 3,272,714 concerne la production industrielle d'ARN à partir de levures cultivées dans un milieu dans lequel la concentration en zinc et en acide phosphorique ainsi que le coefficient d'absorption d'oxygène sont contrôlés ; le procédé décrit ne permet pas de produire spécifiquement un ARN d'intérêt mais uniquement une quantité importante de l'ensemble des ARN endogènes de levure (ARN totaux de levure) ;
- la Demande de brevet européen 0 317 209 concerne la production industrielle de protéines hétérologues. Pour augmenter le niveau d'expression d'un gène hétérologue d'intérêt chez la levure, il est préconisé de remplacer les souches classiques de levure transformées par un vecteur d'expression du gène d'intérêt selon les protocoles standards de transformation cytoplasmique (chlorure de lithium), par des souches de levures déficientes pour la respiration obtenues par délétion de tout ou partie du génome mitochondrial (souches *rho*⁰ et *rho*⁻), transformées par ce même vecteur. Lesdites levures transformées qui sont déficientes pour la respiration sont cultivées dans un milieu contenant une source de carbone fermentescible (glucose). Même si ce système décrit pour la production de protéines était transposé à la production d'ARN, il ne permettrait pas de produire spécifiquement un ARN d'intérêt mais uniquement un mélange d'ARN endogènes de levure et d'ARN exogènes d'intérêt dans lequel la proportion d'ARN d'intérêt dans la fraction d'ARN totaux de levure, est augmentée.

La Demanderesse s'est en conséquence donné pour but de pourvoir à un procédé de production d'ARN qui ne présente pas les inconvénients des procédés habituellement utilisés et qui réponde ainsi mieux aux besoins de la pratique, en ce qu'il permet de synthétiser *in vivo,* dans un système cellulaire, n'importe quel ARN d'intérêt en grandes quantités ou avec un rendement élevé et à un coût significativement inférieur à celui obtenu avec les méthode *in vitro* de l'art antérieur.

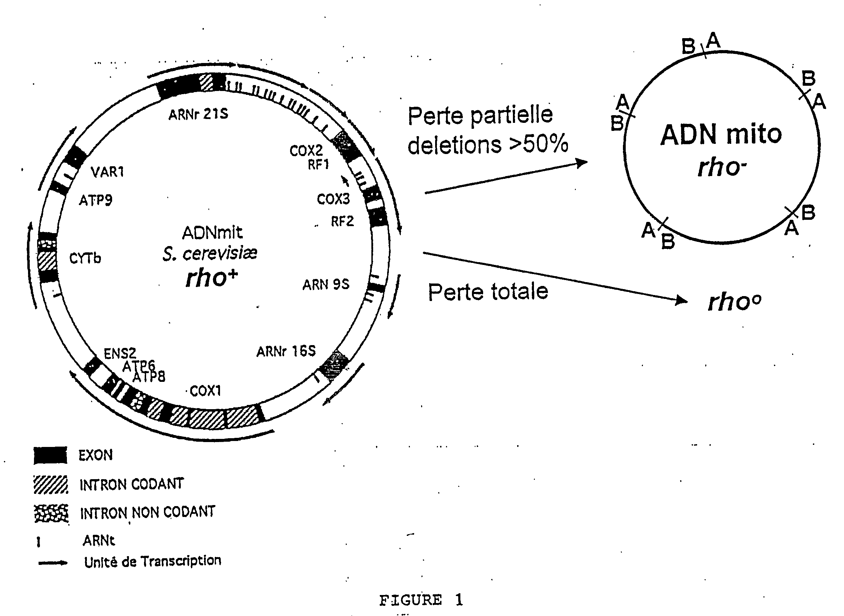

Les mitochondries sont des organelles cellulaires possédant une information génétique propre ; l'ADN mitochondrial de levure contient les gènes de quelques protéines nécessaires à la fonction respiratoire des mitochondries, ainsi que quelques gènes nécessaires au fonctionnement de l'appareil de synthèse protéique mitochondrial (figure 1). Des méthodes de transformation mitochondriale (méthode de bombardement biolistique) ont été mises au point chez la levure, dans le but d'analyser la fonction des gènes mitochondriaux et la régulation de leur expression (Jonhston et al., Science, 1988, 240, 1538; Bonnefoy et al., Methods in Enzymology, 2001, 350, 97, 111 ; Anziano et Butow, P.N.A.S., 1991, 88, 5592-5596). Dans une première étape, une souche de levure dépourvue d'ADN mitochondrial (souche *rho*⁰) est bombardée avec un ADN exogène adsorbé sur des microprojectiles métalliques, de façon à produire une souche *rho⁻* synthétique ; l'ADN exogène, généralement un plasmide, contient tout ou partie du gène à analyser et un fragment d'un marqueur ou rapporteur (gène de la chaîne respiratoire permettant la croissance en milieu non-fermentescible). Dans une seconde étape, les levures *rho⁻* synthétiques ayant incorporé l'ADN exogène sont identifiées par croisement avec des levures testrices *rho*⁺ comprenant une mutation dans ledit gène marqueur ou rapporteur, et isolement des levures *rho⁻* synthétiques aptes à produire des diploïdes capables de croître en milieu non-fermentescible.

La souche *rho⁻* synthétique ainsi identifiée est croisée avec une souche de levure adaptée à l'étude dudit gène mitochondrial (souche portant une mutation dans ledit gène ou souche de type sauvage (génétique inverse)) et l'effet en cis (recombinaison homologue) ou en trans du gène mitochondrial est analysé dans les levures résultant du croisement.

Les Demanderesses ont montré que des souches de levure *rho*⁰ peuvent avantageusement être transformées par un ADN codant pour un ARN hétérologue d'intérêt (ARN non codé par un gène mitochondrial) et utilisées pour produire l'ARN d'intérêt dans leurs mitochondries. Cet ARN est facilement isolé sous forme stable et en grande quantité, à partir des mitochondries de la souche *rho*⁻synthétique, dans la mesure où les seuls ARN produits dans les mitochondries de ladite souche *rho⁻* synthétique sont ceux qui sont codés par l'ADN utilisé pour la transformation.

La présente invention a en conséquence pour objet, un procédé de production d'un ARN hétérologue d'intérêt, lequel procédé est caractérisé en ce qu'il comprend au moins les étapes suivantes :
(1) la transformation des mitochondries de cellules de levure, notamment de *S. cerevisiae,* dépourvues d'ADN mitochondrial (souche *rho*⁰) avec un vecteur de transcription mitochondriale comprenant l'ADN codant ledit ARN hétérologue d'intérêt sous le contrôle d'élément(s) régulateur(s) de la transcription mitochondriale, et un gène rapporteur de la transformation mitochondriale ou un fragment dudit gène rapporteur ; le procédé selon l'invention permet ainsi la transcription de toute séquence hétérologue d'ADN quelle que soit son origine intra ou inter-spécifique (y compris les ADNs mitochondriaux d'autres organismes que la levure ou les ADNs chloroplastiques), ainsi que des séquences d'ADN synthétiques n'existant pas dans la nature ; on obtient ainsi un transformant mitochondrial ou une souche *rho⁻* synthétique.
(2) l'identification des transformants mitochondriaux de levure ayant incorporé l'ADN d'intérêt,
(3) la culture des transformants mitochondriaux de levure sélectionnés à l'étape (2), de préférence en phase exponentielle de croissance,
(4) l'isolement des mitochondries, à partir des transformants mitochondriaux de levure cultivés selon l'étape (3) et
(5) l'extraction et la purification de l'ARN hétérologue d'intérêt, à partir desdites mitochondries.

Dans les conditions du procédé selon l'invention, l'ARN obtenu est stable.

Le procédé de l'invention permet de produire simultanément un ou plusieurs ARN d'intérêt à partir du vecteur de transcription mitochondriale.

### Définitions (voir figures 1 à 3)

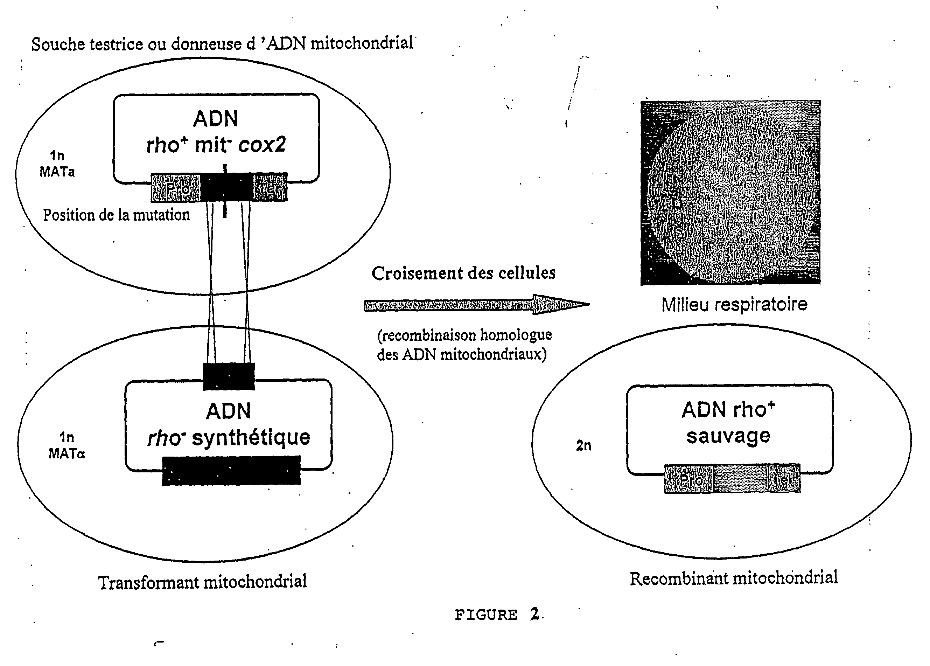

- les termes «cellule de levure», «souche de levure», «cellule», « levure » et « souche » sont considérés comme équivalents dans le contexte de la présente invention et employés indifféremment ; il en va de même pour les souches de levure *rho*⁺, *rho*⁰ et *rho⁻* telles que définies ci-après.
- ARN hétérologue d'intérêt : tout ARN naturel ou synthétique, non-codé par le génome mitochondrial de levure.
- Souche *rho*⁺ : souche de levure comprenant un ADN mitochondrial intact et fonctionnel comme dans des souches sauvages, ou un ADN mitochondrial portant des altérations locales dans sa séquence, de type mit-, qui inactivent la fonction respiratoire des mitochondries. (figure 1).
- Souche *rho*⁰ : souche de levure dépourvue d'ADN mitochondrial, caractérisée par une incapacité de croissance dans un milieu contenant une source de carbone non fermentescible et une absence de synthèse protéique mitochondriale (figure 1).
- Souche *rho*⁻ : souche de levure comprenant une large délétion, toujours supérieure à 50 %, du génome mitochondrial. La région du génome mitochondrial conservée est réitérée, directement ou en palindrome, de manière à reconstituer une masse d'ADN mitochondrial équivalente à celle existant dans une cellule sauvage de levure. Les délétions de type *rho*- entraînent toujours avec elles au moins un des gènes mitochondriaux nécessaire aux synthèses protéiques mitochondriales, étant donné que ces gènes sont répartis uniformément le long du génome mitochondrial. Les souches *rho*⁻ sont donc phénotypiquement équivalentes à des souches *rho*⁰ avec la perte de toute activité de synthèse protéique mitochondriale et donc l'inactivation totale de la fonction respiratoire de l'organelle (figure 1).
- Souche *rho⁻* synthétique : souche de levure initialement *rho⁰* dont les mitochondries ont été transformées par un ADN exogène (ADN hétérologue), notamment par la méthode de bombardement biolistique de cellules de levure précitée. Cette transformation est rendue possible par la capacité des cellules de levure de pouvoir répliquer et maintenir n'importe quel fragment d'ADN, notamment un vecteur bactérien (plasmide). Pour les raisons énumérées ci-dessus, aucune protéine ne peut être synthétisée dans les mitochondries de cellules *rho*⁻ synthétiques. En revanche, la machinerie de transcription mitochondriale est fonctionnelle dans ces cellules. Ainsi, l'introduction d'un fragment d'ADN portant un gène d'intérêt sous la dépendance des séquences signal de transcription mitochondriale, dans des mitochondries d'une cellule *rho*⁰, permet d'obtenir une souche de levure exprimant dans ses mitochondries uniquement l'ARN dudit gène mais pas la protéine correspondante. Comme dans une souche *rho*⁻ naturelle, l'ADN introduit artificiellement dans les mitochondries sera réitéré de manière à produire dans l'organelle une masse d'ADN équivalente à celle présente dans des mitochondries de souche sauvage *rho*⁺. Le gène d'intérêt sera donc présent dans la souche *rho*⁻ synthétique en un nombre relativement élevé de copies (plus de 3000 pour un gène d'intérêt d'environ 1kb). C'est donc pour ces propriétés proches de celles des cellules *rho⁻* (naturelles) que de telles cellules sont appelées par analogie cellules *rho*⁻ synthétiques (figure 2).
- Souche mit⁻ : souche de levure comprenant une altération locale (substitution nucléotidique, courte délétion ou insertion) dans la séquence d'un gène mitochondrial qui code pour une des sous unités du système énergétique mitochondrial comme le gène *COXII* ou *COX2.*
- Transformants mitochondriaux : transformants obtenus notamment par bombardement des cellules de levure, selon la méthode biolistique précitée. Le bombardement d'une souche *rho*⁰ conduit à l'obtention de transformants mitochondriaux qui sont des *rho*⁻ synthétiques. Tout vecteur peut être utilisé pour les bombardements, mais pour identifier les transformants mitochondriaux, il faut un vecteur comprenant un marqueur du génome mitochondrial de la levure, par exemple le gène *COX2,* ou un fragment dudit gène.
- Recombinants mitochondriaux : ils sont obtenus par recombinaison homologue après mise en présence de la souche *rho*⁻ synthétique avec une souche *rho*⁺.
- Marqueur d'auxotrophie : mutation dans un gène connu de la voie de biosynthèse ou d'utilisation d'un acide aminé, d'un nucléotide, d'un substrat carboné, etc ...

De manière surprenante, le procédé selon l'invention :
- est facilement industrialisable (utilisation de fermenteurs classiques) et
- permet effectivement d'obtenir l'ARN d'intérêt en quantités importantes, pour un faible coût, après une purification aisée à mettre en place.

En outre, il présente les avantages suivants :
- le fait qu'il inclue une synthèse *in vivo,* dans des levures, de l'ARN d'intérêt, lui permet de bénéficier de tous les contrôles de qualité cellulaire, notamment : (1) une fidélité de transcription maximale, minimisant considérablement les risques d'erreur d'incorporation, par plusieurs ordres de grandeur par rapport aux procédés existants, et (2) une absence de modifications post transcriptionnelles qui ont pour effet de changer la séquence de l'ARN par rapport à la séquence de son gène, du fait qu'il n'y pas d'édition de l'ARN dans les mitochondries de levure,
- les coûts de fabrication sont essentiellement indépendants de la longueur de l'ARN. Ils sont essentiellement constitués par l'achat de réactifs peu onéreux (milieux de culture et produits pour la purification de l'ARN), alors que les procédés de l'Art antérieur utilisent des réactifs très onéreux (nucléotides, kits enzymatiques) en grandes quantités.,

Un tel procédé constitue donc une alternative particulièrement avantageuse aux procédés de production d'ARN *in vitro,* selon l'art antérieur, économiquement et du point de vue de la qualité des molécules fabriquées.

Conformément à l'invention, préalablement à l'étape (1), ledit ADN codant l'ARN d'intérêt peut être amplifié, notamment par PCR, puis il est cloné dans ledit vecteur de transcription mitochondriale. Dans un tel cas, les amorces oligonucléotidiques sont établies de la manière suivante : l'oligonucléotide P1 est complémentaire de la région 5' de l'ADN d'intérêt adjacente au +1 de la transcription. Il comprend avantageusement un site de restriction permettant le clonage de l'ADN amplifié dans le vecteur de transformation et éventuellement un site facilitant la purification de l'ARN d'intérêt. L'oligonucléotide P2 est, lui, complémentaire de la région 3' de l'ADN d'intérêt adjacente au stop de la transcription. Là aussi, l'oligonucléotide comprend avantageusement un site de restriction pour le clonage de l'ADN amplifié et éventuellement des séquences facilitant la purification de l'ARN d'intérêt. Les séquences facilitant la purification de l'ARN d'intérêt sont notamment des séquences complémentaires d'oligonucléotides couplées à une phase solide ; par exemple une séquence oligodA permet de purifier l'ARN-oligoA ainsi obtenu, à l'aide d'un oligodT couplé notamment à des billes magnétiques. Avantageusement, lesdites séquences facilitant la purification de l'ARN d'intérêt sont des séquences clivables.

Selon un mode de mise en oeuvre avantageux dudit procédé, ladite séquence d'ADN codant l'ARN d'intérêt est sous le contrôle d'un promoteur et d'un terminateur de transcription fonctionnels dans les mitochondries de levure. Parmi les séquences régulatrices utilisables on peut citer de façon non-limitative les séquences signal d'expression de gènes mitochondriaux tels que *COX1* et *COX2.*

Selon un autre mode de mise en oeuvre avantageux du procédé selon l'invention, ledit gène rapporteur de la transformation mitochondriale est un gène codant pour l'une des protéines de la chaîne respiratoire de levure [gènes de l'apocytochrome b et des sous-unités I, II et III de la cytochrome-oxydase (*COX*)] ; ledit gène correspondant à une unité de transcription fonctionnelle incluant le promoteur, la séquence codante et le terminateur de transcription.

Lorsqu'un vecteur contenant un gène rapporteur de la transformation mitochondriale est mis en oeuvre, de préférence un vecteur d'origine bactérienne (plasmide), par exemple le plasmide pUC18, seuls deux ARNs sont donc produits dans le système selon l'invention : l'ARN du gène rapporteur (*COX2*) et l'ARN d'intérêt. Il est donc aisé de séparer ces deux ARNs de par leurs tailles respectives, par exemple par électrophorèse, HPLC, RMN, affinité, etc...En outre, ce gène rapporteur permet la complémentation d'un allèle mit⁻ du gène rapporteur présent dans la souche testrice de la transformation, soit en trans (transcomplémentation), soit par recombinaison homologue.

Selon un autre mode de mise en oeuvre avantageux du procédé selon l'invention, ledit vecteur de transformation mitochondriale contient un fragment d'un gène rapporteur de la transformation mitochondriale tel que défini ci-dessus. Lorsque le gène rapporteur (*COX2*) est remplacé par un fragment de ce même gène qui n'est pas transcrit, la purification de l'ARN d'intérêt est plus aisée étant donné que ledit ARN d'intérêt se retrouve le seul et unique ARN présent dans les mitochondries. En outre, ce fragment de gène rapporteur permet la complémentation par recombinaison homologue d'un allèle mit⁻ dudit gène rapporteur présent dans la souche testrice de la transformation.

Le vecteur de transformation mitochondriale tel que défini ci-dessus peut en outre être amélioré de la manière suivante : en introduisant (i) des séquences permettant une plus grande stabilité du gène d'intérêt par exemple par l'ajout d'une séquence Ori (origine de réplication de l'ADN mitochondrial) de levure telle que *S. cerevisiae,* de manière à accroître l'efficacité de réplication du vecteur dans les mitochondries et/ou (ii) des séquences facilitant la purification de l'ARN telles que définies ci-dessus et éventuellement des séquences permettant d'éliminer les séquences de maturation de l'ARN.

Selon un autre mode de mise en oeuvre avantageux du procédé selon l'invention, la transformation selon l'étape (1) comprend l'adsorption dudit vecteur de transcription mitochondriale sur des microprojectiles métalliques (tungstène ou or) et la projection desdits microprojectiles sur lesdites cellules, de manière connue en soi, conformément par exemple au procédé de biolistique tel que décrit dans l'article de N. Bonnefoy et al., (Methods in Enzymology, 2001, 350, 97-111). L'appareil utilisé est par exemple un système PDS-1000/He (BioRad). Cet instrument utilise une onde de choc à l'hélium pour l'accélération de particules microscopiques en direction d'un tapis de cellules sur une boîte de Pétri. Ces particules font une taille de 0,45 µm de diamètre, ce qui représente environ 10 % du diamètre d'une cellule de levure. Dans un nombre limité de cellules, ces microprojectiles traversent la paroi de la levure et atteignent la mitochondrie. Les mitochondries des cellules *rho*⁰ ne contenant pas d'ADN propre, l'ADN introduit par biolistique est le seul ADN présent dans ces organites et l'on obtient ainsi des cellules *rho*⁻ synthétiques.

Selon un autre mode de mise en oeuvre avantageux du procédé selon l'invention, les cellules de levure dépourvues d'ADN mitochondrial sont celles d'une souche *rho*⁰*.* A titre d'exemple non-limitatif, on peut citer les souches *rho*⁰ suivantes dérivées de la souche DBY947 : ATCC 201440 (MCC109*rho*⁰ [MATa, ade2-101, ura3-52, karl-1 (*rho*⁰)]), ATCC 201442 (MCC123*rho*⁰[MATa, ade2-101, ura3-52, karl-1 (*rho*⁰)]) et DFS160*rho*⁰ (M.E. Sanchirico et al., EMBO J., 1998, 17, 19, 5796-5804. d ; Steele et al., PNAS, 1996, 93, 5253-5257).

Selon un autre mode de mise en oeuvre avantageux du procédé selon l'invention, lesdites cellules de levure dépourvues d'ADN mitochondrial sont modifiées de telle sorte que les gènes codant pour des protéines de dégradation de l'ARN dans la mitochondrie sont inactivés (modifiés ou délétés). On connaît à ce jour plusieurs protéines mitochondriales, toutes d'origine nucléaire, qui interviennent dans le *turn-over* des ARNs mitochondriaux, comme par exemple Suv3p et une sous-unité d'une exoribonucléase 3'-5' (DSS1p). En utilisant des souches de levure dans lesquelles les gènes de ces protéines ont été inactivés (souches Δ*SUV3* ou ΔD*SS1*) comme notamment les souches ΔSUV3Δi et ΔDSSΔi (Dziembowski et al., J. Biol. Chem., 2003, 278, 1603-1611) ou les souches ATCC n°4002799, 4012799, 4022799 et 4032799, on peut accroître la stabilité des ARNs synthétisés dans la mitochondrie, selon le procédé de l'invention.

Selon un autre mode de mise en oeuvre avantageux du procédé selon l'invention, lesdites cellules de levure dépourvues d'ADN mitochondrial comprennent, une copie d'un gène codant pour une ARN polymérase exogène et incluant une séquence d'adressage mitochondrial, intégrée dans leur chromosome (copie chromosomique).

Les séquences d'ARN polymérase exogènes fonctionnelles en cellules de levure et les séquences d'adressage mitochondrial sont connues ; elles sont introduites dans le chromosome de levure par recombinaison homologue, selon les techniques classiques connues de l'Homme du métier.

La majorité des protéines mitochondriales sont d'origine nucléaire. C'est le cas en particulier pour la machinerie protéique nécessaire à la transcription de l'ADN en ARN. Cette machinerie est donc importée vers la mitochondrie et reste fonctionnelle même lorsque les cellules sont *rhp*⁰ et donc dépourvues d'ADN mitochondrial. Par contre, une partie de la machinerie de traduction de l'ARN en protéine est codée par le génome mitochondrial. Par conséquent, si le génome mitochondrial est absent -c'est le cas dans les cellules *rho⁻* synthétiques, l'ADN étranger introduit dans la mitochondrie n'est pas traduit en protéine. Dans ce contexte, l'ADN introduit par biolistique est le seul ADN présent dans les mitochondries des cellules *rho⁻* synthétiques obtenues. Il sera transcrit en ARN mais cet ARN ne sera pas traduit en protéine.

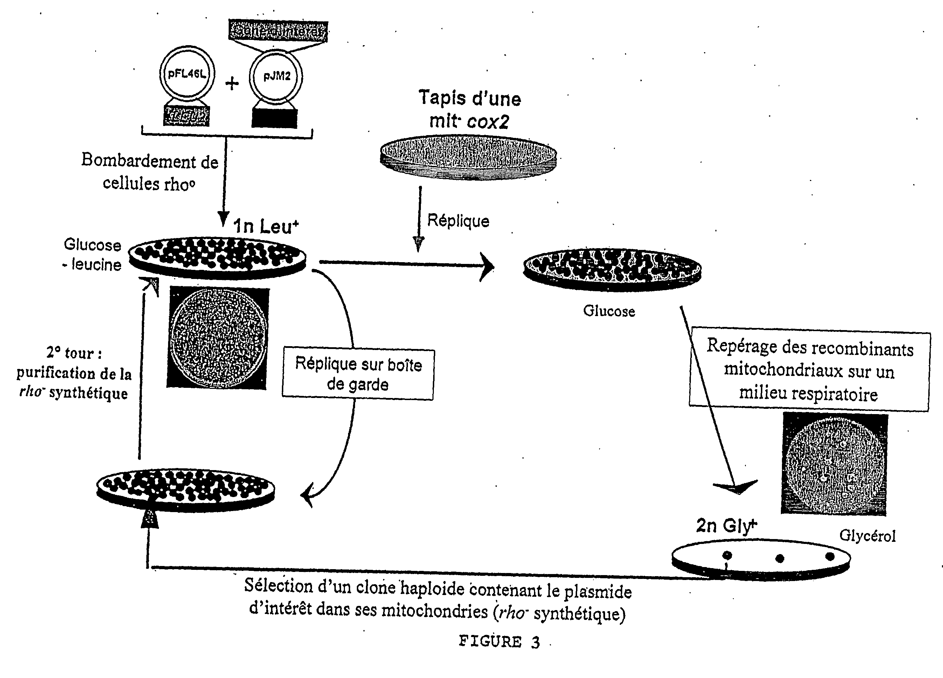

Selon un autre mode de mise en oeuvre avantageux dudit procédé, l'étape (1) comprend la co-transformation de la levure avec ledit vecteur de transcription mitochondriale et un vecteur réplicatif chez la levure comprenant un marqueur de sélection nucléaire, comme un marqueur d'auxotrophie des cellules transformées tel que *LEU2,* ledit vecteur est par exemple pFL46L (ATCC n° 77210) ou Yep351 (ATCC n° 37672) (figure 3). Le gène sauvage porté par le plasmide vient ainsi complémenter fonctionnellement le gène muté porté par les cellules transformées.

Selon un autre mode de mise en oeuvre avantageux du procédé selon l'invention, l'étape (2) comprend :
(a₀) le croisement des levures transformées *rho*⁻ synthétiques, obtenues à l'issue de l'étape (1), avec une souche testrice de levure de type *rho*⁺ mit⁻, pour faciliter l'identification desdites cellules transformées, ladite souche testrice contenant une altération locale (substitution nucléotidique (mutation ponctuelle), courte délétion ou insertion) dans une région du génome mitochondrial correspondant au gène rapporteur de la transformation mitochondriale ou au fragment de ce gène utilisé à l'étape (1), par exemple l'un des gènes de la chaîne respiratoire de levure tel que *COX2* ; la séquence sauvage correspondante (gène ou fragment de gène) est portée par le vecteur de transcription mitochondriale utilisé pour transformer la mitochondrie de la souche hôte *rho*⁰ à l'étape (1),
(b₀) l'identification des transformants mitochondriaux (cellules *rho*⁻ synthétiques) qui donnent, une fois croisés, des cellules diploïdes, capables de croître sur un milieu non fermentescible : seules les cellules de la souche hôte qui contiennent dans leurs mitochondries le vecteur de transcription mitochondriale portant le gène d'intérêt et l'allèle sauvage de la mutation mit⁻ présente dans l'ADN mitochondrial de la souche testrice donneront, après recombinaison des ADNs mitochondriaux parentaux, des cellules diploïdes recombinantes *rho*⁺ mit⁺ qui seront mises en évidence par leur capacité à croître sur un milieu non fermentescible (milieu respiratoire), après réplique au velours des diploïdes sur un tel milieu. Sur ce milieu, par exemple, dans le cas où le vecteur de transcription mitochondriale contient un fragment de gène rapporteur, ni les parents du croisement (souche hôte *rho*⁰ et souche testrice *rho*⁺ mit⁻), ni les diploïdes non-recombinants *rho*⁺ mit⁻ issus de ce croisement ne seront capables de croître. Cette étape permet de cerner sur la boîte initiale obtenue après bombardement de la souche hôte *rho*⁰ des zones où se trouvent des cellules de cette souche hôte dont les mitochondries ont acquis le vecteur de transcription mitochondriale portant le gène d'intérêt et
(c₀) la répétition dudit croisement jusqu'à obtention de colonies de levures isolées, identifiées comme étant des transformants mitochondriaux porteurs du vecteur de transformation mitochondriale (cellules *rho*⁻ synthétiques).

De manière plus précise, les transformants nucléaires obtenus après bombardement sont croisés avec une souche qui elle possède un ADN mitochondrial *rho*⁺ mais également une mutation mit⁻ qui l'empêche de croître sur un milieu respiratoire ; la séquence sauvage correspondante est présente dans le gène rapporteur ou le fragment de gène rapporteur porté par le vecteur de transcription mitochondriale compris dans la *rho*⁻ synthétique. De cette façon, après croisement, l'ADN mitochondrial muté et la séquence sauvage correspondante sur la *rho*⁻ synthétique vont être en présence. La recombinaison homologue permettra alors de corriger la mutation mit⁻ et les diploïdes obtenus retrouveront une croissance respiratoire, d'où le terme de « marker rescue ». En pratique : on considère 1000 à 10000 transformants nucléaires répartis sur la boîte après bombardement parmi lesquels on veut identifier ceux qui sont aussi des transformants mitochondriaux. On réplique donc cette boîte au velours (pour garder la même disposition des clones sur la boîte de Pétri) sur une boîte du même milieu (boîte de garde) et sur un tapis de la souche testrice *rho*⁺ mit⁻. Après croisement cette dernière est répliquée sur un milieu respiratoire pour repérer les clones qui ont permis le sauvetage par le marqueur ou « marker rescue ». On revient alors à la boîte de garde (non croisée, réplique de la boîte originelle) et on récupère les clones *rho⁻* synthétiques haploïdes correspondants car ce sont eux qui sont sélectionnés *in fine* (figure 3).

En d'autres termes, les deux souches qui sont croisées à l'étape (a₀) ne peuvent pas croître sur un milieu non fermentescible. Il sera donc facile de repérer les clones recombinants qui ont recouvré la capacité de croître sur ce type de milieu. Il n'est donc pas nécessaire de sélectionner les diploïdes sur un milieu spécifique (au niveau des marqueurs d'auxotrophie) avant de répliquer les croisements sur un milieu non fermentescible. Seules les cellules de la souche hôte qui contiennent dans leurs mitochondries le vecteur de transcription mitochondriale pourront donner, après croisement, des clones pouvant croître sur un milieu non fermentescible.

Plusieurs cas sont possibles : diploïdes recombinants mais aussi diploïdes non recombinants si le gène marqueur est entier dans le vecteur (complémentation en *trans* par l'ARN produit par l'ADN de la *rho*⁻ synthétique), et enfin cytoductants recombinants ou non (la mutation kar 1-1 d'une des 2 souches retarde la fusion des noyaux et permet d'obtenir après croisement des haploïdes qui ont quand même subi une fusion des cytoplasmes et donc des mitochondries).

A l'étape (c₀), la purification des transformants mitochondriaux est effectuée en prélevant sur la boîte de garde (non croisée) la zone dans laquelle on a repéré un clone qui donne, après croisement, une croissance sur un milieu non fermentescible et en répétant le croisement après réétalement des cellules en colonies individuelles ; cette étape (c₀) permet donc de sélectionner, au deuxième ou au troisième tour, une colonie d'un transformant mitochondrial pur. En effet, le problème est que parmi 5000 clones sur une seule boîte, on ne peut pas être sûr d'avoir récupéré un clone pur mais seulement une zone mélangée à partir de laquelle il est préférable de purifier la *rho⁻* synthétique. Pour ceci on étale la zone récupérée en colonies individuelles et on refait le même croisement testeur. Après environ 3 purifications successives, on obtient un clone pur *rho*⁻ synthétique.

En variante, l'étape (2) comprend :
(a₁) une première sélection ou présélection des cellules de levure à l'aide du marqueur nucléaire tel que défini ci-dessus, par culture dans un milieu approprié,
(b₁) une deuxième sélection à partir des cellules de levure sélectionnées en (a₁), conformément aux étapes (a₀), (b₀ et (c₀), telles que définies ci-dessus.

Par exemple, une fois les cellules *rho*⁰ bombardées, elles sont incubées à 28°C, température optimale de croissance de la levure. Dans un premier temps, les cellules sont incubées sur un milieu sélectif dépourvu de l'acide aminé ou du nucléotide correspondant au marqueur d'auxotrophie desdites cellules. Cette mutation rend la croissance desdites cellules dépendante de l'ajout dans le milieu de culture par exemple de l'acide aminé qui ne peut plus être synthétisé. Pour identifier les transformants mitochondriaux, les levures capables de croître sur milieu sélectif sont croisées avec une souche de signe sexuel opposé. Cette souche est mit⁻, son ADN mitochondrial est présent, mais le gène *COX2* a été délété. Les diploïdes sont sélectionnés sur un milieu à source de carbone non-fermentescible. Seules les levures dont les mitochondries ont été transformées par le plasmide portant *COX2* peuvent former des diploïdes viables avec la souche mit⁻ *COX2-* sur ce type de milieu (complémentation par recombinaison d'ADN ou en *trans* par l'ARN « sauvage » de *COX2* dans la *rho-* synthétique). Ce croisement est alors répété jusqu'à obtention de colonies isolées identifiées comme étant des transformants mitochondriaux capables de produire l'ARN d'intérêt dans leurs mitochondries (figure 3).

De manière préférée et conformément à l'invention, après croisement avec une souche portant une mutation mit⁻, par exemple dans le gène *COX2,* la complémentation de cette mutation peut se faire transitoirement en *trans* par traduction de l'ARN messager du gène sauvage (*COX2)* apporté par la souche *rho⁻* synthétique, après fusion des réseaux mitochondriaux parentaux. Ce croisement constitue un test pour identifier et sélectionner les transformants d'intérêt en vue de la production de l'ARN d'intérêt.

En d'autres termes, après le bombardement des cellules *rho*⁰, on procède tout d'abord et préférentiellement à la sélection des transformants nucléaires. Ils sont nombreux (5 000 par boîte de Pétri), de sorte que les colonies auxquels ils donnent naissance ne sont pas bien séparées les unes des autres. Donc dans un premier temps, les croisements avec la souche testrice (par une réplique au velours) permettront de cerner sur la boîte de tir originale des régions contenant des transformants mitochondriaux (en général une dizaine par boîte). Les cellules de cette zone seront diluées et réétalées sur un milieu solide, pour cette fois obtenir des colonies bien séparées. Ces colonies individuelles sont à nouveau testées par croisement avec la souche mit⁻ testrice de manière à identifier celles issues de cellules contenant dans leurs mitochondries l'ADN d'intérêt.

Selon un autre mode de mise en oeuvre du procédé selon l'invention, l'isolement des mitochondries, conformément à l'étape (4) du procédé selon l'invention, comprend avantageusement, après lyse ou broyage desdites cellules, un isolement des mitochondries selon des méthodes connues, notamment en gradient de saccharose ou selon la méthode décrite dans Guérin B. et al., Methods Enzymol., 1979, 55, 149-59.

De manière préférée, l'étape (4) du procédé selon l'invention comprend avantageusement après lyse ou broyage desdites cellules, l'isolement des mitochondries par au moins deux étapes de centrifugation appropriées, à des vitesses de préférence comprises entre 750 g et 12.500 g, et la récupération du dernier culot de centrifugation.

Conformément à l'invention, la purification de l'ARN mitochondrial selon l'étape (5) est effectuée par des techniques classiques. Elle peut-être réalisée selon le protocole décrit dans di Rago JP. et al., J. Biol. Chem., 1988, 263, 12564-12570 ou un protocole modifié dans lequel l'étape d'extraction et de purification de l'ARN d'intérêt par extractions phénoliques successives est remplacée par une étape de purification de l'ARN par adsorption sur une résine appropriée, suivie du décrochage de l'ARN dans une solution appropriée, notamment dans l'eau.

Selon un autre mode de mise en oeuvre du procédé selon l'invention, la purification de l'ARN selon l'étape (5) comprend :
- l'élimination des acides nucléiques contaminants en particulier de nombreux ARNs fixés en périphérie de la mitochondrie, en présence de tampons appropriés, le premier tampon comprenant au moins un chélateur d'ions bivalents comme l'EDTA et l'EGTA et le deuxième tampon comprenant une RNase et éventuellement une DNase,
- la lyse des mitochondries, en présence d'au moins un détergent, un chélateur d'ions bivalents et dans une zone de pH entre 7 et 8 ; à titre d'exemple, on peut citer le tampon suivant : SDS 1 %, EDTA 10 mM et Tris/HCl pH 7,5 ; et
- l'isolement et la purification de l'ARN d'intérêt par tout moyen approprié.

L'ARN ainsi purifié en solution aqueuse est dosé, analysé sur gel d'agarose et séquencé.

Ledit ARN peut être utilisé tel quel ou subir des étapes supplémentaires de modification, en fonction de l'utilisation envisagée. Parmi ces modifications qui sont réalisées selon les techniques classiques, on peut citer de façon non-limitative : la coupure des séquences de maturation ; des modifications chimiques, notamment pour augmenter la stabilité de l'ARN *in vivo,* hybridation en double brin, notamment pour former un siRNA, digestion pour obtenir des fragments de petites tailles couvrant tout le gène.

La présente invention a également pour objet une souche de levure dépourvue d'ADN mitochondrial comprenant une copie chromosomique d'un gène codant pour une ARN polymérase exogène et incluant une séquence d'adressage mitochondrial. De préférence, ladite souche est obtenue à partir de cellules *rho*⁰ et/ou Δ*SUV3* ou Δ*DSS1*, telles que définies ci-dessus.

La présente invention a également pour objet, un transformant mitochondrial de levure dépourvue d'ADN mitochondrial tel que défini ci-dessus. De préférence, ledit transformant est obtenu à partir d'une souche *rho*⁰ et/ou Δ*SUV3* ou Δ*DSS1*, et/ou comprenant une ARN polymérase exogène, telle que définie ci-dessus.

La présente invention a également pour objet l'utilisation de transformants mitochondriaux de cellules de levure dépourvues d'ADN mitochondrial tels que définis ci-dessus, pour la production industrielle d'un ARN hétérologue d'intérêt.

La présente invention a également pour objet un système utile pour la production industrielle d'ARN hétérologue d'intérêt présélectionné, caractérisé en ce qu'il comprend :
- des cellules de levure transformées au moins par un vecteur de transcription mitochondriale (cellules *rho*⁻ synthétiques) comprenant l'ADN codant l'ARN hétérologue d'intérêt sous le contrôle d'élément(s) régulateur(s) de la transcription mitochondriale et un gène rapporteur de la transformation mitochondriale ou un fragment dudit gène rapporteur,
- au moins un milieu de culture convenable permettant de sélectionner lesdites cellules transformées (transformants mitochondriaux),
- des cellules de levure testrices, de type *rho*⁺ mit⁻,
- des fermenteurs et des milieux de culture appropriés et
- des tampons appropriés pour isoler les mitochondries à partir des cellules *rho*⁻ synthétiques et en extraire l'ARN d'intérêt.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 illustre l'ADN mitochondrial de *S. cerevisiae* ; l'ADN mitochondrial code notamment pour : 3 sous-unités de l'ATP synthase (6, 8, 9) ; 4 sous-unités de la chaîne respiratoire ; 2 ARNr + 1 protéine du mitoribosome ; 24 ARNt ;
- la figure 2 illustre le principe du sauvetage par le marqueur ou « marker rescue » ;
- la figure 3 représente la construction d'une souche *rho*⁻ synthétique ;
- la figure 4 illustre l'analyse en Northem blot de l'ARN mitochondrial de levures *rho⁻* synthétiques, transformées par un vecteur de transcription mitochondriale comprenant le gène *RIP1.*
   Il doit être bien entendu, toutefois, que ces exemples sont donnés uniquement à titre d'illustration de l'objet de l'invention, dont ils ne constituent en aucune manière une limitation.

### EXEMPLE : Production de l'ARN du gène PIP1 selon le procédé de l'invention A) Matériel et méthodes

### 1) Vecteur de transcription mitochondriale

pJM2 (pTZ18-U, avec *COX2* sauvage; YIN J. et al., Tsinghua Science and Technology, 1999, 4, 2 ; Bio-Rad) dans lequel a été cloné le gène *RIP1*^{m}, version en code mitochondrial du gène *RIP1* codant pour une sous-unité du complexe III de la chaîne respiratoire flanqué des séquences signal d'expression de *COX1.*

### 2) Vecteur navette comprenant un marqueur de sélection nucléaire

Yep352 (2 µ, URA3) (ATCC n° 37673)

### 3) Transformation de mitochondries de cellules de S. cerevisiae dépourvues d'ADN mitochondrial avec les vecteurs tels que décrits en 1) et 2)

Le dérivé *rho*⁰ de la souche W303-1B (Matα, ade2, trp1, his3, leu2, ura3), dénommé W303-1B (ATCC n° 201238)/A/50, est cotransformé avec les vecteurs tels que définis en 1) et 2), selon la méthode de biolistique décrite dans l'article de N. Bonnefoy et al., (Methods in Enzymology, 2001, 350, 97-111), à l'aide d'un appareil Biolistic PDS-1000/He (BioRad).

### 4) Méthode d'identification des cellules transformées

Dans un premier temps, on procède à la sélection des transformants nucléaires sur un milieu synthétique dépourvu d'uracile. Les transformants mitochondriaux sont identifiés parmi les transformants nucléaires URA3⁺ par leur capacité à produire des diploïdes avec une croissance sur milieu non fermentescible après croisement avec la souche testrice TF145 (*MATα, ade2*) (Speno H. et al., J. Biol. Chem., 1995, 270, 43, 25363-25369). Cette souche n'est pas capable de croître en milieu non-fermentescible (milieu respiratoire) du fait qu'elle comprend une délétion dans le gène mitochondrial *COX2* (*cox2-17*).

### 5) Conditions de culture des cellules de levures :

Les transformants mitochondriaux sont cultivés en fermenteurs jusqu'en milieu de phase exponentielle de croissance dans un milieu riche contenant du galactose comme source de carbone.

### 6) Méthode de purification des mitochondries et de l'ARN :

### 6.1) Isolement des mitochondries

Les transformants mitochondriaux de levure sont cultivés en fermenteur jusqu'en milieu de phase exponentielle de croissance soit une DO entre 3 et 4. Ils sont alors récoltés et lysés ou broyés et leurs mitochondries sont purifiées par des méthodes classiques.

Brièvement, le protocole mis en oeuvre est le suivant :
Les mitochondries des transformants mitochondriaux sont isolées et purifiées après digestion de leur paroi cellulaire avec de la zymolyase dans un milieu (1,35 M sorbitol) protégeant osmotiquement l'intégrité des cellules débarrassées de leur paroi (appelées sphéroplastes). Les sphéroplastes sont soumis à un choc osmotique dans un tampon préservant l'intégrité des mitochondries (0,6 M sorbitol). Les débris cellulaires (noyaux, parois) sont éliminés par plusieurs (au minimum 2) centrifugations à basse vitesse (750 g) du lysat des sphéroplastes. Le dernier surnageant est centrifugé à haute vitesse (12 500 g) pour culoter les mitochondries.
De manière plus précise, les conditions sont les suivantes :

### I / Récolte des levures

Les levures sont centrifugées à basse vitesse (4°C, 10 minutes à 3800 *g*).

### II / 2 Lavages des levures à l'eau distillée glacée

Les culots sont alors repris dans de l'eau distillée fraîche (4°C).

On centrifuge à 4°C, pendant 5 min à 3800 g. On élimine le surnageant et on lave une deuxième fois à l'eau distillée, puis une nouvelle centrifugation est effectuée dans les mêmes conditions.

### III / Fragilisation de la paroi cellulaire

Le 2-mercaptoéthanol rompt les ponts disulfures entre les différentes mannoprotéines de la paroi, facilitant l'action ultérieure de la zymolyase. A partir de la D.O. à 600 nm de la culture, on évalue le poids sec de levure au moyen de la formule suivante :

PS (g) = 0,28 DO Volume_culture (L)

On incube dans un volume de 20 ml de tampon/g de PS.

On reprend donc le culot dans un tampon de pré-incubation « SH » (2-mercaptoéthanol 0,5 M, Tris-Base 0,1 M, pH 9,3) et on incube alors pendant 10 min à 30°C avec agitation.

### IV /Lavages des levures au tampon de lavage au KCl

Le but de ces lavages est d'éliminer toutes traces d'agent réducteur.

On ajoute le tampon KCl (KCl 0,5 M, Tris-Base 10 mM, pH 7,0) au tampon de pré-incubation SH. On centrifuge à 4°C, pendant 5 min à 3800 g. On élimine le surnageant. On effectue ainsi 2 lavages successifs au tampon KCl.

### V / Digestion à la Zymolyase à 30°C de la paroi cellulaire

La zymolyase détruit la paroi cellulaire.

La paroi des levures est constituée d'un squelette de chitine auquel s'ajoutent d'autres protéines.

Pour digérer la paroi, une possibilité est d'utiliser la zymolyase produite par la bactérie *Arthrobacter luteus* ou un mélange enzymatique (cytohélicase) : enzyme de suc gastrique de l'escargot (*Helix pomatia*), donnant alors des protoplastes de levure.

On reprend le culot avec la solution de digestion, à raison de 10 à 15 mg de zymolyase/10 ml de tampon de digestion (sorbitol 1,35 M, EGTA 1 mM, acide citrique 10 mM, phosphate disodique 30 mM, pH 5,8).

On arrête la digestion à la zymolyase quand 80 à 90% des cellules sont digérées, en complétant avec le tampon de lavage des protoplastes au KCl.

On centrifuge alors pendant 5 min à 12 500 g.

### VI /Lavages des protoplastes

Le culot est repris rapidement dans le tampon de lavage des protoplastes (ou sphéroplastes = cellules débarrassées de leur paroi) (sorbitol 0,75 M, mannitol 0,4 M, BSA 0,1 %, Tris-Maléate 10 mM, pH 6,8). On centrifuge pendant 5 min à 12 500 g. Le surnageant est éliminé, puis on effectue un deuxième lavage du culot.

### VII / Homogénéisation et broyages

On reprend les culots dans quelques ml du tampon d'homogénéisation (mannitol 0,6 M, EGTA 2 mM, BSA 0,2 %, Tris-Maléate 10 mM, pH 6,8). On verse la préparation dans un potter. On descend et remonte le potter une dizaine de fois ; on mixe le préparation à vitesse modérée, on récupère un maximum de la préparation puis on redistribue le tout en plusieurs tubes.

### VIII l Isolement des mitochondries par centrifugation différentielle

On centrifuge à basse vitesse (750 g) pendant 8 min, à 4°C. On conserve les surnageants. Le culot peut éventuellement être repris dans le tampon d'homogénéisation, centrifugé et le surnageant ajouté au précédent surnageant.

On centrifuge à plus haute vitesse (12 500 g) pendant 10 min, à 4°C, puis on élimine les surnageants.

Les culots sont repris dans le tampon de récupération (mannitol 0,6 M, EGTA 2 mM, Tris-Maléate 10 mM, pH 6,8).

On centrifuge à basse vitesse (8 min, 750 g à 4°C), puis on centrifuge les sumageants à haute vitesse (10 min, 12 500 g, à 4°C). On élimine le surnageant et on refait éventuellement un troisième cycle de centrifugation basse vitesse/haute vitesse.

### IX / Récupération finale des mitochondries

Le culot de mitochondries est repris dans un volume minimum de tampon de récupération (Mannitol, EGTA, Tris-Maléate pH 6,8, tel que défini ci-dessus), puis passé au potter pour l'homogénéiser.

### X / Rendement

Pour 2 litres de culture, on produit environ 2-4 g de levures.

Une préparation de mitochondries à la zymolyase permet d'obtenir 1 à 1,5 ml de mitochondries à une concentration de 30 mg/ml de protéines mitochondriales soit 30 à 45 mg de protéines.

### 6.2) Extraction des ARN

### a) méthode A

Les mitochondries sont dans un premier temps incubées en présence d'EDTA et d'EGTA pour les débarrasser des polysomes et des ARNs situés en périphérie de la mitochondrie. Puis elles sont lavées en tampon dépourvu d'EDTA et d'EGTA et incubées dans ce même tampon en présence de RNase et de DNase afin de les débarrasser des ARN cytoplasmiques situés en périphérie des mitochondries et des reliquats d'ADN nucléaire. L'action des RNase et DNase est interrompue par centrifugation à 12 500 g. L'extraction de l'ARN d'intérêt est alors réalisée selon le protocole d'extraction d'ARN mitochondrial décrit dans Di Rago et al., 1988, précité. Le culot mitochondrial est lavé en tampon contenant de l'EDTA et de l'EGTA et lysé en présence de SDS 1 %, EDTA 10 mM et Tris HCl pH 7,5. Les acides nucléiques sont purifiés par extractions phénolyques successives. La phase aqueuse finale est éventuellement lavée par action de chloroforme::acide isoamylique, puis les acides nucléiques sont précipités. A cette étape, l'ADN mitochondrial est éventuellement dégradé par ajout de DNase. L'ARN ainsi purifié est dosé, analysé sur gel d'agarose et séquencé.

De manière plus précise, le protocole d'extraction d'ARN mitochondrial est le suivant :
Le tampon de récupération (défini ci-dessus) a été additionné de 10 mM EDTA ; les différentes centrifugations sont effectuées à 4°C.
   - Centrifugation 12 500 g 10'
   - Récupération dans du tampon de récupération, sans EDTA ni EGTA
   - Centrifugation 12 500 g 10'
   - Reprise dans du tampon de récupération sans EDTA ni EGTA. Ajout de RNase et de DNase et incubation de 15' à 37°C
   - Centrifugation 12 500 g 10'
   - Lavages dans du tampon de récupération avec EDTA et EGTA, suivis de centrifugations
   - Reprise dans le tampon de Lyse (SDS 2%, EDTA 10 mM, Tris HCl 10 mM, pH 7,5) et ajout du même volume de mélange phénol :chloroforme :alcool isoamylique 49 :49 :2. Vortexer 3', laisser reposer 2' à 4°C, revortexer 2-3'.
   - Centrifugation 5' à 8 000 g et à 4°C.
   - Prélèvement de la phase aqueuse et ajout du mélange phénol/chloroforme, environ 5 fois.
   - Ajout à la dernière phase aqueuse de 0,3 M d'acétate de sodium pH 5,2 et 2,5 volumes d'éthanol 100%. Laisser 15' à -80°C puis centrifuger 20' à 8 000 g à 4°C et laver le culot.
   - Reprise du culot dans 3 ml de tampon MgCl₂ 10mM, Tris/acétate 20mM, pH 7,4 contenant 75 µl de complexe vanadyl-ribonucléosides (inhibiteur de RNase) 200mM et 2 µl de DNase sans RNase (dans 50% de glycérol à une concentration de 0,5 mg/ml).
   - Élimination de la DNase avec un volume phénol/chloroforme/alcool isoamylique 49:49:2, puis un volume de chloroforme/alcool isoamylique 24 : 1, puis un volume de diéthyléther saturé en eau.
   - Précipitation des ARN de la phase aqueuse par ajout de 0,3 M d'acétate de sodium et 2,5 volumes d'éthanol 100%.
   - Reprise du culot dans 50 à 100 µl d'eau stérile.
   - Dosage et vérification des ARN puis préparation et purification supplémentaire si besoin.

### b) méthode B

Les mitochondries sont dans un premier temps incubées en présence d'EDTA, d'EGTA et de RNAse pour débarrasser totalement l'échantillon des des ARNs cytosoliques normalement accrochés à la périphérie externe du compartiment mitochondriale. L'extraction des ARN situés à l'intérieur des mitochondries est alors réalisée avec un kit approprié disponible dans le commerce (Rneasy Protect Starter Kit™, QIAGEN, Cat. No. 74121). Dans cette procédure, l'échantillon mitochondrial est lysé avec un tampon spécifique suivi immédiatement de la purification des ARN qu'il contient en une seule étape par adsorption des seuls ARN de l'échantillon sur une résine appropriée suivie du décrochage des ARN avec de l'eau . L'ARN ainsi purifié est dosé, analysé sur gel d'agarose et séquencé.

### B) Résultats

Les ARNs mitochondriaux purifiés sont analysés par la technique Northern blot avec une sonde ADN spécifique du gène *RIP1*, marquée au P³² et, à titre de contrôle, une sonde ADN spécifique du gène mitochondrial endogène *COX1* également radiomarquée.

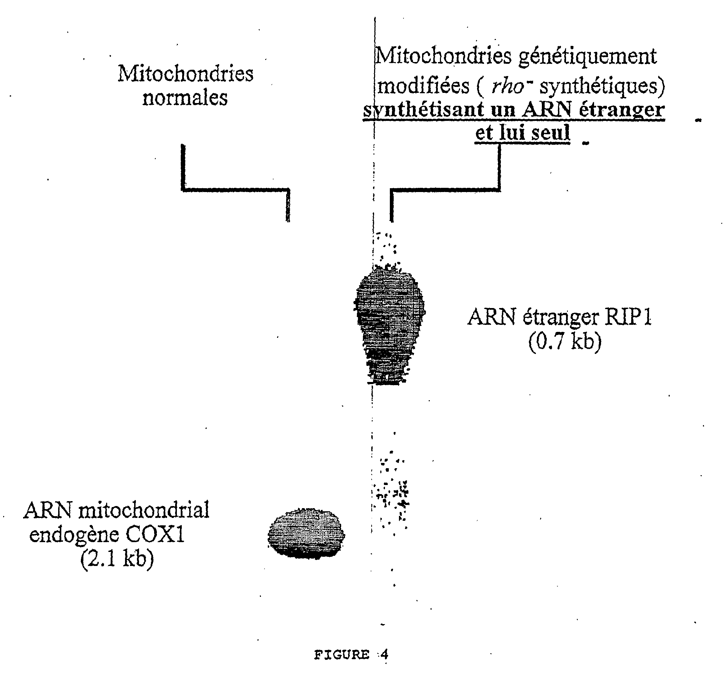

Le signal avec la sonde RIP1 et l'absence de signal avec la sonde *COX1* démontrent que les mitochondries de la souche *rho*⁻ synthétique contiennent l'ARN d'intérêt mais pas d'ARN mitochondrial endogène (figure 4). Par comparaison, on observe un signal avec la sonde *COX1* mais pas avec la sonde RIP1, avec les ARNs mitochondriaux d'une souche sauvage de levure *rho*⁺ mit⁺.

Ces résultats démontrent donc que les mitochondries de la souche *rho*⁻ synthétique sont capables de produire spécifiquement l'ARN d'intérêt

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Procédé de production d'un ARN hétérologue d'intérêt, lequel procédé est **caractérisé en ce qu'**il comprend au moins les étapes suivantes :
(1) la transformation des mitochondries de cellules de levure dépourvues d'ADN mitochondrial avec un vecteur de transcription mitochondriale comprenant au moins une copie de l'ADN codant ledit ARN hétérologue d'intérêt sous le contrôle d'élément(s) régulateur(s) de la transcription mitochondriale, et un gène rapporteur de la transformation mitochondriale ou un fragment dudit gène rapporteur,
(2) l'identification des transformants mitochondriaux de levure ayant incorporé l'ADN d'intérêt,
(3) la culture des transformants mitochondriaux de levure sélectionnés à l'étape (2),
(4) l'isolement des mitochondries, à partir des transformants mitochondriaux de levure obtenus à l'étape (3) et
(5) l'extraction et la purification de l'ARN hétérologue d'intérêt, à partir desdites mitochondries.

2. Procédé selon la revendication 1, **caractérisé en ce que** lesdites cellules de levure dépourvues d'ADN mitochondrial sont des cellules *rho*⁰.

3. Procédé selon la revendication 1 ou la revendication 2, **caractérisé en ce que** lesdites cellules dépourvues d'ADN mitochondrial sont obtenues à partir d'une souche Δ*SUV3* ou Δ*DSS1.*

4. Procédé selon l'une quelconque des revendications 1 à 3, **caractérisé en ce que** lesdites cellules dépourvues d'ADN mitochondrial comprennent une copie chromosomique d'un gène codant pour une ARN polymérase exogène et incluant un signal d'adressage mitochondrial.

5. Procédé selon l'une quelconque des revendications 1 à 4, **caractérisé en ce que** ledit ADN codant l'ARN d'intérêt est sous le contrôle d'un promoteur et d'un terminateur de transcription fonctionnels dans les mitochondries de levure.

6. Procédé selon l'une quelconque des revendications 1 à 5, **caractérisé en ce que** ledit gène rapporteur de la transformation mitochondriale est un gène codant pour l'une des protéines de la chaîne respiratoire de levure.

7. Procédé selon l'une quelconque des revendications 1 à 6, **caractérisé en ce que** ledit vecteur de transcription mitochondriale comprend la séquence d'une origine de réplication de l'ADN mitochondrial.

8. Procédé selon l'une quelconque des revendications 1 à 7, **caractérisé en ce que** le fragment du gène rapporteur de la transformation mitochondriale est un fragment non-transcrit dudit gène rapporteur.

9. Procédé selon l'une quelconque des revendications 1 à 8, **caractérisé en ce que** la transformation selon l'étape (1) comprend l'adsorption dudit vecteur de transcription mitochondriale sur des microprojectiles métalliques et la projection desdits microprojectiles sur lesdites cellules.

10. Procédé selon l'une quelconque des revendications 1 à 9, **caractérisé en ce que** l'étape (1) comprend la co-transformation desdites cellules de levure avec ledit vecteur de transcription mitochondriale et un vecteur réplicatif chez la levure comprenant un marqueur de sélection nucléaire.

11. Procédé selon la revendication 10, **caractérisé en ce que** ledit marqueur nucléaire est un marqueur d'auxotrophie desdites cellules transformées.

12. Procédé selon l'une quelconque des revendication 1 à 11, **caractérisé en ce que** l'étape (2) comprend :
(a₀) le croisement des transformants mitochondriaux de levure obtenus à l'étape (1) avec une souche testrice de levure de type *rho*⁺ mit⁻,
(b₀) l'identification des transformants mitochondriaux qui donnent, une fois croisés, des cellules diploïdes, capables de croître sur un milieu non fermentescible et
(c₀) la répétition dudit croisement jusqu'à obtention de colonies de levures isolées, identifiées comme étant des transformants mitochondriaux porteurs du vecteur de transformation mitochondriale.

13. Procédé selon la revendication 10 ou la revendication 11, **caractérisé en ce que** l'étape (2) comprend :
(a₁) une première sélection ou présélection des cellules de levure à l'aide dudit marqueur nucléaire, par culture dans un milieu approprié,
(b₁) une deuxième sélection à partir des cellules de levure sélectionnées en (a₁), conformément aux étapes (a₀), (b₀ et (c₀), telles que définies à la revendication 12.

14. Procédé selon l'une quelconque des revendications 1 à 13, **caractérisé en ce que** l'isolement des mitochondries, conformément à l'étape (4) du procédé, comprend la lyse ou le broyage desdites cellules puis au moins deux étapes de centrifugation, à des vitesses de préférence comprises entre 750 g et 12.500 g et la récupération du dernier culot de centrifugation.

15. Procédé selon l'une quelconque des revendications 1 à 14, **caractérisé en ce que** l'étape (5) comprend avantageusement :
- l'élimination des acides nucléiques contaminants en présence de tampons appropriés, le premier tampon comprenant au moins un chélateur d'ions bivalents et le deuxième tampon comprenant une RNase et éventuellement une DNase,
- la lyse des mitochondries, en présence d'au moins un détergent, un chélateur d'ions bivalents et dans une zone de pH entre 7 et 8, et
- l'isolement et la purification de l'ARN d'intérêt.

16. Cellule de levure modifiée, **caractérisée en ce qu'**elle est dépourvue d'ADN mitochondrial et **en ce qu'**elle comprend une copie chromosomique d'un gène codant pour une ARN polymérase exogène et incluant un signal d'adressage mitochondrial.

17. Cellule de levure modifiée, **caractérisée en ce qu'**elle est dépourvue d'ADN mitochondrial et **en ce que** ses mitochondries sont transformées par un vecteur de transcription mitochondriale comprenant au moins une copie de l'ADN codant un ARN hétérologue d'intérêt sous le contrôle d'élément(s) régulateur(s) de la transcription mitochondriale, et un fragment non-transcrit d'un gène rapporteur de la transformation mitochondriale, tel que défini à la revendication 7.

18. Cellule de levure modifiée selon la revendication 16 ou la revendication 17, **caractérisée en ce qu'**elle est obtenue à partir d'une souche Δ*SUV3* ou Δ*DSS1.*

19. Cellule de levure modifiée selon l'une quelconque des revendications 16 à 18, **caractérisée en ce qu'**elle est obtenue à partir d'une souche *rho*⁰*.*

20. Utilisation de transformants mitochondriaux de levures dépourvues d'ADN mitochondrial, pour la production industrielle d'un ARN hétérologue d'intérêt.

21. Système utile pour la production industrielle d'un ARN hétérologue d'intérêt, **caractérisé en ce qu'**il comprend :
- des cellules de levure dépourvues d'ADN mitochondrial, notamment de souche *rho*⁰, transformées au moins par un vecteur de transcription mitochondriale tel que défini aux revendications 1 et 5 à 8,
- au moins un milieu de culture convenable permettant de sélectionner lesdites cellules transformées,
- des cellules testrices de levure, de type *rho*⁺ mit⁻,
- des fermenteurs et des milieux de culture appropriés et
- des réactifs appropriés pour isoler les mitochondries à partir de cellules *rho*⁻ synthétiques et en extraire l'ARN hétérologue d'intérêt.

## Claims

1. A method for producing a heterologous RNA of interest, which method is **characterized in that** it comprises at least the following steps:
(1) transforming the mitochondria of yeast cells lacking mitochondrial DNA with a mitochondrial transcription vector comprising at least one copy of the DNA encoding said heterologous RNA of interest under the control of regulatory element(s) for mitochondrial transcription, and a mitochondrial transformation reporter gene or a fragment of said reporter gene;
(2) identifying the yeast mitochondrial transformants that have incorporated the DNA of interest;
(3) culturing the yeast mitochondrial transformants selected in step (2);
(4) isolating the mitochondria from the yeast mitochondrial transformants obtained in step (3), and
(5) extracting and purifying the heterologous RNA of interest from said mitochondria.

2. The method as claimed in claim 1, **characterized in that** said yeast cells lacking mitochondrial DNA are *rho⁰* cells.

3. The method as claimed in claim 1 or claim 2, **characterized in that** said cells lacking mitochondrial DNA are obtained from a Δ*SUV3* or Δ*DSS1* strain.

4. The method as claimed in any one of claims 1 to 3, **characterized in that** said cells lacking mitochondrial DNA comprise a chromosomal copy of a gene encoding an exogenous RNA polymerase and including a mitochondrial targeting signal.

5. The method as claimed in any one of claims 1 to 4, **characterized in that** said DNA encoding the RNA of interest is under the control of a promoter and a transcription terminator that are functional in yeast mitochondria.

6. The method as claimed in any one of claims 1 to 5, **characterized in that** said mitochondrial transformation reporter gene is a gene encoding one of the proteins of a yeast respiratory chain.

7. The method as claimed in any one of claims 1 to 6, **characterized in that** said mitochondrial transcription vector comprises the sequence of an origin of replication of the mitochondrial DNA.

8. The method as claimed in any one of claims 1 to 7, **characterized in that** the mitochondrial transformation reporter gene fragment is an untranscribed fragment of said reporter gene.

9. The method as claimed in any one of claims 1 to 8, **characterized in that** the transformation according to step (1) comprises the adsorption of said mitochondrial transcription vector onto metal microprojectiles and the projection of said microprojectiles onto said cells.

10. The method as claimed in any one of claims 1 to 9, **characterized in that** step (1) comprises the cotransformation of said yeast cells with said mitochondrial transcription vector and a vector that is replicative in yeast, comprising a nuclear selection marker.

11. The method as claimed in claim 10, **characterized in that** said nuclear marker is an auxotrophic marker of said transformed cells.

12. The method as claimed in any one of claims 1 to 11, **characterized in that** step (2) comprises:
(a₀) crossing the yeast mitochondrial transformants obtained in step (1) with a yeast tester strain of *rho⁺* mit⁻ type,
(b₀) identifying the mitochondrial transformants which, once crossed, give diploid cells capable of growing on a non-fermentable medium, and
(c₀) repeating said crossing until isolated yeast colonies identified as being mitochondrial transformants carrying the mitochondrial transformation vector are obtained.

13. The method as claimed in claim 10 or claim 11, **characterized in that** step (2) comprises:
(a₁) a first selection or preselection of the yeast cells by means of said nuclear marker, by culturing in an appropriate medium,
(b₁) a second selection from the yeast cells selected in (a₁), in accordance with steps (a₀), (b₀) and (c₀), as defined in claim 12.

14. The method as claimed in any one of claims 1 to 13, **characterized in that** the isolation of the mitochondria, in accordance with step (4) of the method, comprises lysis or grinding of said cells, and then at least two centrifugation steps, at speeds preferably of between 750 g and 12 500 g, and recovery of the final centrifugation pellet.

15. The method as claimed in any one of claims 1 to 14, **characterized in that** step (5) advantageously comprises:
- eliminating the contaminating nucleic acids in the presence of appropriate buffers, the first buffer comprising at least one divalent ion-chelating agent, and the second buffer comprising an RNase and, optionally, a DNase,
- lysing the mitochondria in the presence of at least one detergent and a divalent ion-chelating agent and within a pH range of between 7 and 8, and
- isolating and purifying the RNA of interest.

16. A modified yeast cell, **characterized in that** it lacks mitochondrial DNA and **in that** it comprises a chromosomal copy of a gene encoding an exogenous RNA polymerase and including a mitochondrial targeting signal.

17. A modified yeast cell, **characterized in that** it lacks mitochondrial DNA and **in that** its mitochondria are transformed with a mitochondrial transcription vector comprising at least one copy of the DNA encoding the heterologous RNA of interest under the control of regulatory element(s) for mitochondrial transcription as defined in claim 7.

18. The modified yeast cell as claimed in claim 16 or claim 17, **characterized in that** it is obtained from a Δ*SUV3* or Δ*DSS1* strain.

19. The modified yeast cell as claimed in any one of claims 16 to 18, **characterized in that** it is obtained from a *rho⁰* strain.

20. The use of yeast mitochondrial transformants lacking mitochondrial DNA, for the industrial production of a heterologous RNA of interest.

21. A system for carrying out the industrial production of a heterologous RNA of interest, **characterized in that** it comprises:
- yeast cells lacking mitochondrial DNA, in particular of *rho⁰* strain, transformed with at least one mitochondrial transcription vector as defined in claims 1 and 5 to 8,
- at least one suitable culture medium for selecting said transformed cells,
- yeast tester cells of *rho⁺* mit⁻ type,
- appropriate fermenters and culture media, and
- appropriate reagents for isolating the mitochondria from synthetic *rho⁻* cells and extracting the heterologous RNA of interest therefrom.

## Patentansprüche

1. Verfahren zur Herstellung einer heterologen RNA von Interesse, wobei das Verfahren **dadurch gekennzeichnet ist, dass** es wenigstens die folgenden Schritte umfasst:
(1) die Transformation von Mitochondrien von Hefezellen, die frei von mitochondrialer DNA sind, mit einem Vektor zur mitochondrialen Transkription, umfassend wenigstens eine Kopie der DNA, die für die genannte heterologe RNA von Interesse codiert, unter der Kontrolle von (einem) regulatorischen Element(en) der mitochondrialen Transkription, und ein Reportergen der mitochondrialen Transkription oder ein Fragment des Reportergens,
(2) die Identifizierung von mitochondrialen Hefetransformanten, die die DNA von Interesse aufgenommen haben,
(3) die Kultur der mitochondrialen Hefetransformanten, die in Schritt (2) selektiert wurden,
(4) die Isolierung von Mitochondrien aus den mitochondrialen Hefetransformanten, die in Schritt (3) erhalten wurden, und
(5) die Extraktion und die Reinigung der heterologen RNA von Interesse aus den genannten Mitochondrien.

2. Verfahren nach Anspruch 1, **dadurch gekennzeichnet, dass** die genannten Hefezellen, die frei von mitochondrialer DNA sind, *rho⁰*-Zellen sind.

3. Verfahren nach Anspruch 1 oder nach Anspruch 2, **dadurch gekennzeichnet, dass** die genannten Zellen, die frei von mitochondrialer DNA sind, aus einem Stamm Δ*SUV3* oder Δ*DSS1* erhalten werden.

4. Verfahren nach einem der Ansprüche 1 bis 3, **dadurch gekennzeichnet, dass** die genannten Zellen, die frei von mitochondrialer DNA sind, eine chromosomale Kopie eines Gens, das für eine exogene RNA-Polymerase codiert und ein Signal der mitochondrialen Adressierung einschließt, umfassen.

5. Verfahren nach einem der Ansprüche 1 bis 4, **dadurch gekennzeichnet, dass** die genannte DNA, die für die RNA von Interesse codiert, unter der Kontrolle eines Promotors und eines Terminators der Transkription, die in den Mitochondrien von Hefe funktionell sind, steht.

6. Verfahren nach einem der Ansprüche 1 bis 5, **dadurch gekennzeichnet, dass** das genannte Reportergen der mitochondrialen Transformation ein Gen ist, das für eines der Proteine der Atmungskette von Hefe codiert.

7. Verfahren nach einem der Ansprüche 1 bis 6, **dadurch gekennzeichnet, dass** der genannte Vektor zur mitochondrialen Transkription die Sequenz eines Replikationsursprungs der mitochondrialen DNA umfasst.

8. Verfahren nach einem der Ansprüche 1 bis 7, **dadurch gekennzeichnet, dass** das Fragment des Reportergens der mitochondrialen Transformation ein nicht transkribiertes Fragment des Reportergens ist.

9. Verfahren nach einem der Ansprüche 1 bis 8, **dadurch gekennzeichnet, dass** die Transformation nach dem Schritt (1) die Adsorption des Vektors zur mitochondrialen Transkription auf metallischen Mikroprojektilen und die Projektion der Mikroprojektile auf die Zellen umfasst.

10. Verfahren nach einem der Ansprüche 1 bis 9, **dadurch gekennzeichnet, dass** der Schritt (1) die Co-Transformation der Hefezellen mit dem Vektor zur mitochondrialen Transkription und einem bei der Hefe replikativen Vektor, der einen nukleären Selektionsmarker umfasst, umfasst.

11. Verfahren nach Anspruch 10, **dadurch gekennzeichnet, dass** der genannte nukleäre Marker ein Marker der Auxotrophie der transformierten Zellen ist.

12. Verfahren nach einem der Ansprüche 1 bis 11, **dadurch gekennzeichnet, dass** der Schritt (2) umfasst:
(a₀)die Kreuzung von mitochondrialen Hefetransformanten, die im Schritt (1) erhalten wurden, mit einem Teststamm ("souche testrice") von Hefe des Typs *rho⁺* mit⁻,
(b₀)die Identifizierung von mitochondrialen Transformanten, die, wenn sie gekreuzt sind, diploide Zellen ergeben, die dazu befähig sind, auf einem nicht fermentierbaren Medium zu wachsen, und
(c₀)die Wiederholung der Kreuzung bis zum Erhalt von isolierten Hefekolonien, identifiziert als mitochondriale Transformanten, die den Vektor zur mitochondrialen Transformation tragen.

13. Verfahren nach Anspruch 10 oder nach Anspruch 11, **dadurch gekennzeichnet, dass** der Schritt (2) umfasst:
(a₁)eine erste Selektion oder Vorselektion der Hefezellen mit Hilfe des nukleären Markers mittels Kultur in einem geeigneten Medium,
(b₁)eine zweite Selektion, ausgehend von den Hefezellen, die in (a₁) selektiert wurden, gemäß den Schritten (a₀), (b₀) und
(c₀), wie sie in Anspruch 12 definiert sind.

14. Verfahren nach einem der Ansprüche 1 bis 13, **dadurch gekennzeichnet, dass** die Isolierung von Mitochondrien gemäß dem Schritt (4) des Verfahrens die Lyse oder das Aufbrechen der Zellen, dann wenigstens zwei Schritte der Zentrifugation bei Geschwindigkeiten, die vorzugsweise zwischen 750 g und 12.500 g liegen, und die Gewinnung des letzten Sediments der Zentrifugation umfasst.

15. Verfahren nach einem der Ansprüche 1 bis 14, **dadurch gekennzeichnet, dass** der Schritt (5) vorzugsweise umfasst:
- die Elimination von kontaminierenden Nukleinsäuren in Gegenwart von geeigneten Puffern, wobei der erste Puffer wenigstens einen Chelatbildner für zweiwertige Ionen umfasst, und der zweite Puffer eine RNase und gegebenenfalls eine DNase umfasst,
- die Lyse der Mitochondrien in Gegenwart von wenigstens einem Detergens, einem Chelatbildner für zweiwertige Ionen und in einem pH-Bereich zwischen 7 und 8 und
- die Isolierung und die Reinigung der RNA von Interesse.

16. Modifizierte Hefezelle, **dadurch gekennzeichnet, dass** sie frei von mitochondrialer DNA ist und **dadurch**, dass sie eine chromosomale Kopie eines Gens, das für eine exogene RNA-Polymerase codiert und ein Signal zur mitochondrialen Adressierung einschließt, umfasst.

17. Modifizierte Hefezelle, **dadurch gekennzeichnet, dass** sie frei von mitochondrialer DNA ist und **dadurch**, dass ihre Mitochondrien transformiert sind durch einen Vektor zur mitochondrialen Transkription, umfassend wenigstens eine Kopie der DNA, die für eine heterologe RNA von Interesse codiert, unter der Kontrolle eines/von regulatorischen Elements/Elementen der mitochondrialen Transkription, und ein nicht transkribiertes Fragment eines Reportergens der mitochondrialen Transformation, wie im Anspruch 7 definiert.

18. Modifizierte Hefezelle nach Anspruch 16 oder nach Anspruch 17, **dadurch gekennzeichnet dass** sie ausgehend von einem Stamm Δ*SUV3* oder Δ*DSS1* erhalten ist.

19. Modifizierte Hefezelle nach einem der Ansprüche 16 bis 18, **dadurch gekennzeichnet dass** sie ausgehend von einem Stamm *rho⁰* erhalten ist.

20. Verwendung von mitochondrialen Transformanten von Hefen, die frei von mitochondrialer DNA sind, für die industrielle Herstellung einer heterologen RNA von Interesse.

21. System, das für die industrielle Herstellung einer heterologen RNA von Interesse dienlich ist, **dadurch gekennzeichnet, dass** es umfasst:
- Hefezellen, die frei von mitochondrialer DNA sind, insbesondere des Stammes *rho⁰,* transformiert mit wenigstens einem Vektor zur mitochondrialen Transkription, wie in den Ansprüchen 1 und 5 bis 8 definiert,
- wenigstens ein zweckmäßiges Kulturmedium, das es erlaubt, die transformierten Zellen zu selektieren,
- Testzellen von Hefe, vom Typ *rho⁺* mit⁻,
- Fermenter und geeignete Kulturmedien und
- geeignete Reagenzien zum Isolieren der Mitochondrien aus den synthetischen *rho⁻*-Zellen und um ihnen die heterologe RNA von Interesse zu extrahieren.
